# EUROPEAN PATENT APPLICATION

(11) **EP 3 815 635 A1**
(43) Date of publication of application: **05.05.2021**
(21) Application number: 18924328.0
(22) Date of filing: 29.06.2018
(51) Int. Cl.: A61B 17/3207, A61B 17/22, A61M 25/06, A61M 25/10

(54) **CATHETER**

(71) Applicant: ASAHI INTECC CO., LTD., Seto-shi, Aichi 489-0071 (JP)
(72) Inventor: NAKAGAWA, Yuta, Seto-shi, Aichi 489-0071 (JP)
(74) Representative: Winter, Brandl - Partnerschaft mbB
(86) International application number: PCT/JP2018/024798
(87) International publication number: WO 2020/003492

(57) **Abstract**

To provide a catheter capable of preventing tissue pieces attached on the mesh member from falling off when the catheter is removed. A catheter 1 includes a first hollow shaft 10, a tubular mesh member 20 that includes a proximal end joined to a distal end of the first hollow shaft 10 and is formed to expand or contract in a radial direction, a distal end tip 30 that is joined to a distal end of the mesh member 20, a first core wire 50 that includes a distal end joined to the distal end tip 30 and extends inside the mesh member 20 and the first hollow shaft 10 so that a proximal end of the first core wire 50 is positioned on the more proximal end side than the proximal end of the first hollow shaft 10, and a second hollow shaft 60 that slides along an axial direction of the first hollow shaft 10 and is movable between a first position at which the second hollow shaft 60 surrounds an outer periphery of the first hollow shaft 10 with a distal end of the second hollow shaft 60 positioned on the more proximal side than the distal end of the first hollow shaft 10 and a second position at which the second hollow shaft 60 surrounds the outer periphery of the mesh member 20 with the distal end of the second hollow shaft 60 positioned on the more distal end side than the distal end of the first hollow shaft 10.

## Description

### Technical Field

The present invention relates to a catheter.

### Background Art

As a medical instrument for improving a blood flow by removing an occluding object occluding a blood vessel such as chronic total occlusion (CTO), there are known, for example, a medical instrument that expands mesh-formed braided wires in the radial direction at a region with the occluding object in the blood vessel so as to remove the occluded part (see PTL1, for example), or a medical instrument that includes a mesh-formed self-expandable area provided with a cover so as to collect the removed occluding object (see PTL2, for example).

Meanwhile, the occluding object is very hard, and thus it is often difficult to remove the occluding object with the above-described medical instruments. For such a case, there are also proposed the technology of expanding a false lumen using an antegrade guide wire and then guiding a retrograde guide wire through this expanded false lumen and also the technology of expanding a mesh-formed member and receiving the above-described guide wire through the opening of the mesh-formed member (see NPTL1, for example).

### Citation List

### Patent Literature

PTL1: Japanese Patent No. 3655920
PTL2: Japanese Patent Application Laid-open (Translation of PCT Application) No. 2011-517424

### Non-Patent Literature

NPTL1: Shinsuke Nanto, ed., "Revised Edition: Basics and Tips of PCI to Learn For Sure", Yodosha, February 25, 2016, p.222-227

### Summary of Invention

### Technical Problem

However, in a case where the above-described mesh-formed member is expanded, tissue pieces may be attached onto the mesh-formed member, and may fall off in a body (a blood vessel) when the catheter is removed.

In view of the above-described aspects, the present invention aims at providing a catheter capable of preventing tissue pieces attached on the mesh-formed member from falling off when the catheter is removed.

### Solution to Problem

To achieve the above-described object, a catheter according to one form of the invention includes a first hollow shaft, a tubular mesh member that includes a proximal end joined to a distal end of the first hollow shaft and is formed to expand or contract in a radial direction, a distal end tip that is joined to a distal end of the mesh member, a first core wire that includes a distal end joined to the distal end tip and a proximal end extends inside the mesh member and the first hollow shaft so that a proximal end of the first core wire is positioned on the more proximal end side than the proximal end of the first hollow shaft, and a second hollow shaft that slides along an axial direction of the first hollow shaft and is movable between a first position at which the second hollow shaft surrounds an outer periphery of the first hollow shaft with a distal end of the second hollow shaft positioned on the more proximal side than the distal end of the first hollow shaft and a second position at which the second hollow shaft surrounds the outer periphery of the mesh member with the distal end of the second hollow shaft positioned on the more distal end side than the distal end of the first hollow shaft.

The catheter may further include a second core wire that includes a distal end tip joined to the second hollow shaft and a proximal end positioned on the more proximal end side than the proximal end of the first hollow shaft.

The first hollow shaft may include an opening communicating the outside and the inside of the first hollow shaft on the more proximal side than the second hollow shaft located at the first position, and the second core wire may be provided to be inserted into the first hollow shaft through the opening and extended inside the first hollow shaft.

The catheter may further include an inner shaft to which the second core wire is inserted, a protruding portion is provided at the proximal end of the second core wire, and the protruding portion may be formed to be in contact with the proximal end of the inner shaft when the second hollow shaft is located at the second position.

A protruding portion may be provided at the proximal end of the second core wire, and the protruding portion may be formed to be in contact with the proximal end of the first hollow shaft when the second hollow shaft is located at the second position. Advantageous Effects of Invention

The present invention is able to provide a catheter capable of preventing tissue pieces attached on a mesh member from falling off when the catheter is removed. Brief Description of Drawings

FIG. 1 is an overall view of a catheter according to a first embodiment, illustrating a state in which a second hollow shaft is located at a first position.
FIG. 2 is a diagram illustrating a state in which the diameter of a mesh member of FIG. 1 is expanded in diameter.
FIG. 3 is an overall view of the catheter according to the first embodiment, illustrating a state in which the second hollow shaft is located at a second position.
FIG. 4 is an overall view of a catheter according to a second embodiment, illustrating a state in which the second hollow shaft is located at the first position.
FIG. 5 is an overall view of a catheter according to a third embodiment, illustrating a state in which the second hollow shaft is located at the first position.
FIG. 6 is an overall view of a catheter according to a fourth embodiment, illustrating a state in which the second hollow shaft is located at the first position. Description of Embodiments

Hereinafter, the catheters according to the embodiments of the invention are described with reference to the enclosed drawings. However, the invention is not limited to the embodiments illustrated in the drawings.

Note that, in the specification, an "antegrade guide wire" indicates, among guide wires, a guide wire that is pushed toward an operative part such as an occluded part in a blood vessel prior to a catheter, and a "retrograde guide wire" indicates, among guide wires, a guide wire that comes from the distal end side of a catheter in a blood vessel, for example.

Further, in the specification, the "distal end side" indicates a direction along the longitudinal direction of the catheter where the distal end tip is positioned with respect to the mesh member. The "proximal end side" indicates a direction along the above-described longitudinal direction that is the direction opposite to the above-described distal end side. The "distal end" indicates an end portion on the distal end side in each member forming the catheter. The "proximal end" indicates an end portion on the above-described proximal side in each member forming the catheter.

### [First Embodiment]

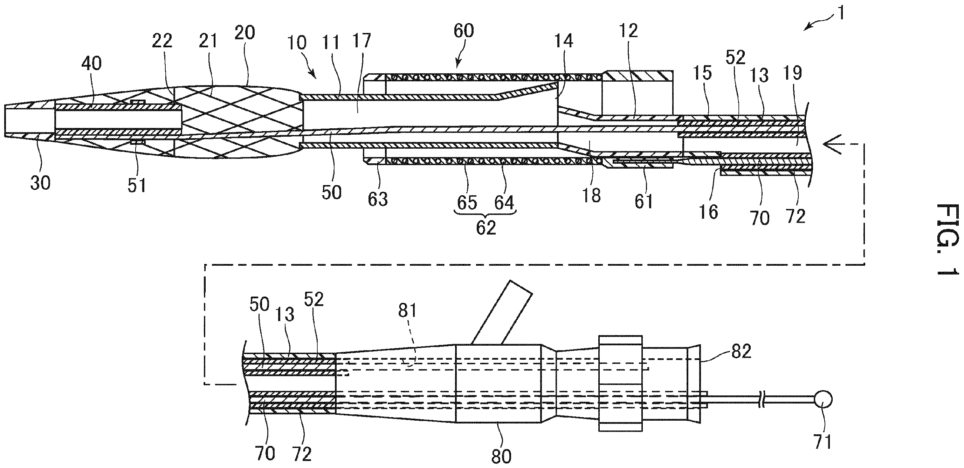

FIG. 1 is an overall view of a catheter 1 according to the first embodiment of the invention, illustrating a state in which a second hollow shaft 60 is located at the first position.

As illustrated in FIG. 1, the catheter 1 includes a first hollow shaft 10, a mesh member 20, a first distal end tip 30, a distal end hollow shaft 40, a first core wire 50, a second hollow shaft 60, a second core wire 70, and a controller 80.

The first hollow shaft 10 includes a distal end side shaft 11, an intermediate shaft 12, and a proximal end side shaft 13. The distal end of the distal end side shaft 11 is connected to the proximal end of the mesh member 20. The distal end of the intermediate shaft 12 is connected to the proximal end of the distal end side shaft 11. At the connection part between the distal end side shaft 11 and the intermediate shaft 12, the distal end side shaft 11 and the intermediate shaft 12 form a guide wire port 14 open toward the proximal end side. A retrograde guide wire is delivered to the outside of the catheter 1 through the guide wire port 14. The distal end of the proximal end side shaft 13 is connected to the proximal end of the intermediate shaft 12. At the distal end portion 15 of the proximal end side shaft 13, there is formed an opening 16 communicating the outer side and the inner side of the first hollow shaft 10. The opening 16 is formed on the more proximal end side than the second hollow shaft 60 located at the first position described later.

The distal end side shaft 11 includes a lumen 17 for inserting therein a retrograde guide wire and the first core wire 50. The intermediate shaft 12 includes a lumen 18 for inserting therein the first core wire 50. The proximal end side shaft 13 includes a lumen 19 for inserting therein the first core wire 50 and the second core wire 70.

The material forming the first hollow shaft 10 is preferably antithrombotic, flexible, and biocompatible because the first hollow shaft 10 is to be inserted into a blood vessel, and may be a resin material or a metal material. The distal end side shaft 11 and the intermediate shaft 12 are required to have flexibility. Thus, a resin material is preferable. For example, there may be adopted polyamide resin, polyolefin resin, polyester resin, polyurethane resin, silicone resin, fluororesin, and the like. The proximal end side shaft 13 is required to have pushability. Thus, a metal tube such as a hypotube may be adopted, for example.

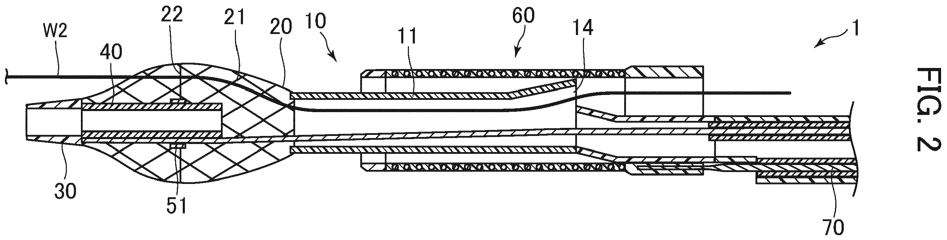

The mesh member 20 is a tubular member that may expand or contract in the radial direction. When the first core wire 50 described later is pulled toward the proximal end side, the mesh member 20 is out-of-plane deformed, protruded radially outward, and thus expanded in diameter as illustrated in FIG. 2. Then, the retrograde guide wire is received in the catheter 1 through the opening of the expanded mesh member 20.

In first embodiment, the mesh member 20 is formed by braiding a plurality of wires 21 in a grid pattern to form a tube shape as a whole. Further, the mesh member 20 has an opening between the adjacent braided wires, and receives the retrograde guide wire through the opening expanded when the mesh member 20 is expanded in diameter. Note that the first distal end tip 30 and the first hollow shaft 10 are joined respectively to the distal end and the proximal end of the wires 21 forming the mesh member 20.

Here, as each of the wires 21 forming the mesh member 20, it is possible to adopt either a single wire or a plurality of wires. The wire 21 may be formed by a strand of a plurality of metal wires having different wire diameters, for example.

A metal material or a resin material may be adopted as the material forming each wire 21 of the mesh member 20. Examples of the resin material include polyamide, polyester, polyarylate, polyetheretherketone, and the like. Note that from the viewpoint of improving the strength and flexibility, a metal material is preferable. Examples of the metal material include stainless steel such as SUS304, a nickel titanium alloy, a cobalt chrome alloy, and the like, for example. Note that each wire may be made of the same material or a different material.

Moreover, the material forming each wire 21 of the mesh member 20 may be a radiopaque material from the viewpoint of improving the visibility of the mesh member 20. Examples of the radiopaque material include gold, platinum, tungsten, an alloy containing these elements (for example, a platinum-nickel alloy), and the like. Note that the radiopaque material may be the combination of the radiopaque material and another material such as a radiopaque material coated on a surface of a non-radiopaque material.

The mesh member 20 is provided with a guide film 22, and the distal end of the guide film 22 is positioned between the proximal end of the first distal end tip 30 and the distal end of the first hollow shaft 10. The guide film 22 smoothly guides the retrograde guide wire received through the opening of the mesh member 20 toward the first hollow shaft 10. The distal end of the guide film 22 is positioned at the substantially center part in the axial direction of the mesh member 20, and the proximal end thereof is positioned at the distal end of the first hollow shaft 10. The guide film 22 is formed on the mesh member 20 so as to crosslink the adjacent wires. Here, the guide film 22 is expanded into a funnel shape when the mesh member 20 is expanded in diameter, whereby the retrograde guide wire is guided into the first hollow shaft 10 through the mesh member 20. Note that the guide film 22 may be in a film form (not illustrated), for example, as long as at least a part thereof (the outer periphery of the distal end of the guide film 22, for example) is joined to the mesh member 20.

Examples of the material forming the guide film 22 include polyethylene, polyurethane, polyamide, polyamide elastomer, polyolefin, polyester, polyester elastomer, and the like, for example. Among these, polyurethane is preferable as the above-described material from the viewpoint of improving the sliding property of the surface. Moreover, the method of forming the guide film 22 is not particularly limited. For example, in the case of the guide film arranged at the mesh member 20, the dip method may be adopted. In the case of the film-formed guide film, there may be adopted a method of fusing the distal end of the film to the mesh member 20, or the like.

The first distal end tip 30 is connected to the distal end of the mesh member 20. Specifically, the first distal end tip 30 is formed to be sharply pointed toward the distal end side so that the catheter 1 easily advances in a blood vessel, and the distal end of each wire of the mesh member 20 and the distal end of the distal end hollow shaft 40 are embedded in the proximal end of the first distal end tip 30.

The material forming the distal end tip 30 preferably has flexibility because the catheter 1 is to advance in a blood vessel. Examples of such a material having flexibility include resin materials such as polyurethane and polyurethane elastomer, for example.

The distal end hollow shaft 40 is connected to the first distal end tip 30 and projects to the proximal end side in the space inside the mesh member 20. As illustrated in FIG. 1, the proximal end of the distal end hollow shaft 40 is positioned between the distal end of the first hollow shaft 10 and the proximal end of the first distal end tip 30 in the space inside the mesh member 20.

The material forming the distal end hollow shaft 40 is preferably anti-thrombotic, flexible, and biocompatible because the distal end hollow shaft 40 is also to be inserted into a blood vessel, similarly to the above-described first hollow shaft 10. Examples of the material include the same materials as those exemplified in the description of the first hollow shaft 10, for example. However, a resin material is preferable from the viewpoint of flexibility.

The distal end of the first core wire 50 is connected to the first distal end tip 30, and the proximal end thereof extends to the controller 80 positioned on the proximal end side of the first hollow shaft 10. Therefore, the first core wire 50 penetrates the mesh member 20 and the lumens 17, 18, and 19 of the first hollow shaft 10. An operator operates the controller 80, whereby the first core wire 50 moves forward or backward in the axial direction, and the mesh member 20 expands or contracts in the radial direction.

The material forming the first core wire 50 preferably has sufficient tensile strength and rigidity from the viewpoint of preventing cutting of the first core wire 50 itself and securely expanding and contracting the mesh member 20. Examples of the material include a metal material such as stainless steel including SUS304, a nickel titanium alloy, a cobalt chrome alloy, and the like.

The first core wire 50 includes a holding member 51 whose cross section is substantially annular or substantially C-shaped. The holding member 51 covers the distal end hollow shaft 40 and the first core wire 50. Therefore, the holding member 51 allows the distal end hollow shaft 40 and the first core wire 50 to move integrally without separation of the proximal end of the distal end hollow shaft 40 from the first core wire 50.

The holding member 51 is made of a resin material such as a polyamide resin, a polyolefin resin, a polyester resin, a polyurethane resin, a silicone resin, and a fluorocarbon resins, or a metal material such as stainless steel including SUS304, a nickel-titanium alloy, and a cobalt chrome alloy. In the case of forming the holding member 51 using a resin, it is preferable to mix a radiopaque material such as bismuth trioxide, tungsten, barium sulfate, or the like into the resin. In the case of forming the holding member 51 using a metal material, it is preferable to use a radiopaque material such as platinum and tungsten. The holding member 51 is preferably positioned near the distal end of the guide film 22 when the mesh member 20 is expanded.

A first inner shaft 52 is fixed in the proximal end side shaft 13. The first inner shaft 52 is hollow-shaped, and extends from the distal end to the proximal end of the proximal end side shaft 13 in the lumen 19. The inner diameter of the first inner shaft 52 is formed to be slightly larger than the outer diameter of the first core wire 50, and the first core wire 50 reciprocatably penetrates the first inner shaft 52. The first inner shaft 52 suppresses the deflection of the first core wire 50.

The material forming the first inner shaft 52 is preferably anti-thrombotic, flexible, and biocompatible because the first inner shaft 52 is to be inserted into a blood vessel, and may be a resin material or a metal material. Examples of the material include the same materials as those exemplified in the description of the first hollow shaft 10.

The second hollow shaft 60 includes a proximal end portion 61, an intermediate portion 62, and a second distal end tip 63.

The proximal end portion 61 is provided to surround the outer periphery of the first hollow shaft 10. The intermediate portion 62 is positioned on the distal end side of the proximal end portion 61, and the proximal end of the intermediate portion 62 is connected to the distal end of the proximal end portion 61. The intermediate portion 62 includes a reinforcing portion 64 and a main body portion 65. The reinforcing portion 64 is formed by braiding a plurality of wires in a grid pattern to form a tube shape as a whole. The main body portion 65 is made of a resin material, and covers the entire of the reinforcing portion 64. Note that the reinforcing portion 64 is not limited to a braided body formed by braiding a plurality of wires in a grid pattern, and may be a coil body of a single wire. Each wire may be a wire formed by twisting a plurality of metal wires. The reinforcing portion 64 improves the pushing force of the second hollow shaft 60.

The second distal end tip 63 is positioned on the distal end side of the intermediate portion 62, and the proximal end of the second distal end tip 63 is connected to the distal end of the intermediate portion 62. The second distal end tip 63 is formed to be sharply pointed toward the distal end side so that it advances easily in a blood vessel.

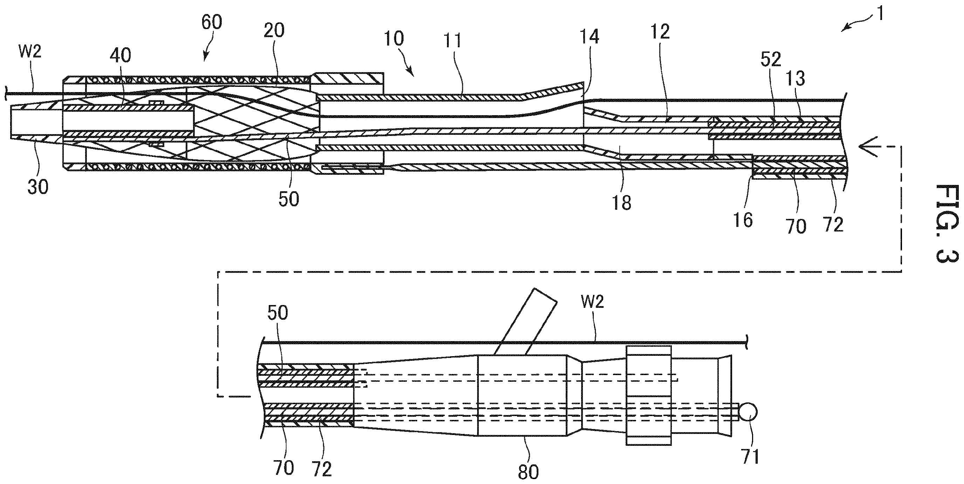

The second hollow shaft 60 is provided slidably along the axial direction (longitudinal direction) of the catheter 1. The second hollow shaft 60 is movably provided between the first position (FIG. 1) at which the second hollow shaft 60 surrounds the outer periphery of the first hollow shaft 10 with the distal end of the second hollow shaft 60 positioned on the more proximal side than the distal end of the first hollow shaft 10, and the second position (FIG. 3) at which the second hollow shaft 60 surrounds the outer periphery of the mesh member 20 with the distal end of the second hollow shaft 60 positioned on the more distal end side than the distal end of the first hollow shaft 10. The length of the second hollow shaft 60 in the axial direction is preferably longer than the length of the mesh member 20 reduced in diameter.

The material forming the proximal end portion 61 is preferably anti-thrombotic, flexible, and biocompatible because the proximal end portion 61 is to be inserted into a blood vessel, and may be a resin material. As the resin material, there may be adopted polyamide resin, polyolefin resin, polyester resin, polyurethane resin, silicone resin, fluororesin, and the like, for example. Examples of the material forming the main body portion 65 include the same materials as those forming the proximal end portion 61, and the like. Examples of the material forming the reinforcing portion 64 include the same materials as those forming the strands 21 of the mesh member 20, and the like. Examples of the material forming the second distal end tip 63 include the same materials as those forming the first distal end tip 30, and the like because the catheter 1 advances in a blood vessel.

The distal end of the second core wire 70 is connected to the proximal end portion 61 of the second hollow shaft 60, and the proximal end thereof extends to be positioned on the more proximal end side than the proximal end of the controller 80. The distal end of the second core wire 70 is connected to be embedded in the proximal end portion 61. The second core wire 70 is provided to be inserted into the proximal end side shaft 13 of the first hollow shaft 10 through the opening 16 of the first hollow shaft 10 and extended in the lumen 19 of the proximal end side shaft 13. A protruding portion 71 is provided at the proximal end of the second core wire 70.

Examples of the material forming the second core wire 70 include the same materials as those forming the first core wire 50.

A second inner shaft 72 is fixed in the proximal end side shaft 13. The second inner shaft 72 is hollow-shaped, and extends from the opening 16 of the proximal end side shaft 13 to the proximal end of the controller 80 in the lumen 19. It is preferable that the distal end and the proximal end of the second inner shaft 72 slightly project from the opening 16 and the proximal end of the controller 80, respectively. The inner diameter of the second inner shaft 72 is formed to be slightly larger than the outer diameter of the second core wire 70, and the second core wire 70 reciprocatably penetrates the second inner shaft 72. The second inner shaft 72 suppresses the deflection of the second core wire 70.

Examples of the material forming the second inner shaft 72 include the same materials as those forming the first inner shaft 52.

An operator moves the second core wire 70 forward or backward, whereby the second hollow shaft 60 slides and moves between the first position and the second position. The protruding portion 71 is formed to be in contact with the proximal end of the second inner shaft 72 in the state where the second hollow shaft 60 is located at the second position. In this manner, when the catheter 1 is removed, it is possible to prevent the second hollow shaft 60 from deviating from the outer periphery of the mesh member 20, and thus prevent tissue pieces attached on the mesh member 20 from falling off.

The controller 80 is a member allowing an operator to hold the catheter 1. The controller 80 is connected to the proximal end of the first hollow shaft 10, and includes a through hole 81 communicating to the lumen 19 of the first hollow shaft 10 and an opening 82 formed on the proximal end of the through hole 81. The second wire 70 and the second inner shaft 72 penetrate the through hole 81. The second inner shaft 72 is fixed in the controller 80.

The following will describe an example of the use mode of the above-described catheter 1.

First, an antegrade guide wire W1 (not illustrated) is, for example, inserted into a blood vessel, and then pushed along the blood vessel to a region where an occluding object exists (hereinafter, also referred to as an "occluded part").

Next, after the distal end of the antegrade guide wire W1 reaches the occluded part, the proximal end of the antegrade guide wire W1 is inserted into the catheter 1 so that it passes through the through hole of the first distal end tip 30, the through hole of the distal end hollow shaft 40, the space inside the mesh member 20, and the lumen 17 of the first hollow shaft 10, and delivered to the outside of the catheter 1 through the guide wire port 14. Then, the distal end of the catheter 1 is pushed to the occluded part in the blood vessel with the antegrade guide wire W1 as a guide. Here, the catheter 1 is inserted into a blood vessel in the state where the mesh member 20 is reduced in diameter, and keeps such a state with a reduced diameter until the distal end of the catheter 1 reaches the occluded part.

After the distal end of the catheter 1 reaches the occluded part, the antegrade guide wire W1 is pulled toward the proximal end side so as to pull out the antegrade guide wire W1 from the catheter 1. Next, the controller 80 is operated to bring back the first core wire 50 toward the proximal end side, whereby a distance between the distal end of the mesh member 20 and the distal end of the first hollow shaft 10 is reduced, and as a result, the mesh member 20 is out-of-plane deformed radially outward. Here, in the first embodiment, the distal end of the guide film 22 is joined to the substantially center part in the axial direction of the mesh member 20. Thus, the diameter of the guide film 22 is expanded following the expanded diameter of the mesh member 20, and the guide film 22 becomes a funnel shape as a whole. Note that the opening of the mesh member 20 is also expanded as the diameter thereof is increased, which allows the state where a retrograde guide wire W2 is received easily. The holding member 51 is preferably positioned around the distal end of the guide film 22 or inside the distal end thereof when the mesh member 20 is expanded.

Next, as illustrated in FIG. 2, the retrograde guide wire W2 coming from the distal end side is received in the catheter 1. The path through which the retrograde guide wire W2 comes may be a false lumen in a blood vessel wall surrounding the occluded part, a through hole penetrating the occluded region, or the like, for example. The retrograde guide wire W2 may come from any of these paths. The retrograde guide wire W2 is received in the inner space of the mesh member 20 through the expanded opening of the mesh member 20, inserted into the distal end shaft 11 of the first hollow shaft 10, and then delivered to the outside of the catheter 1 through the guide wire port 14. The retrograde guide wire W2 delivered from the guide wire port 14 passes through the blood vessel, and then the end of the retrograde guide wire W2 is delivered to the outside of the body.

Next, the controller 80 is operated so as to advance the first core wire 50 toward the distal end side and reduce the diameter of the mesh member 20. The second core wire 70 is advanced toward the distal end side, whereby the second core shaft 60 at the first position is advanced and moved to the second position so as to surround the outer periphery of the mesh member 20, as illustrated in FIG. 3. In this state, the catheter 1 is brought back entirely and removed from the blood vessel. In this manner, the retrograde guide wire W2 is inserted into the blood vessel.

As described above, with the above-described structure of the catheter 1, the retrograde guide wire W2 is received by the mesh member 20, and the mesh member 20 is contracted so that the second hollow shaft 60 is moved from the first position to the second position. This allows the state where the second hollow shaft 60 surrounds the outer periphery of the mesh member 20 before the catheter 1 is removed. The catheter 1 is removed in this state, which makes it possible to prevent tissue pieces attached on the mesh member 20 from falling off.

Further, in the state where the second hollow shaft 60 is located at the second position, the protruding portion 71 is in contact with the proximal end of the second inner shaft 72. Thus, it is possible to suppress deviation of the second hollow shaft 60 from the outer periphery of the mesh member 20, and thus prevent tissue pieces attached on the mesh member 20 from falling off. Moreover, the second inner shaft 72 suppresses the deflection of the second core wire 70.

Further, the second core wire 70 is pushed, whereby the second hollow shaft 60 is moved from the first position to the second position. Therefore, the operator is able to easily move the second hollow shaft 60, and it is possible to prevent tissue pieces attached on the mesh member 20 from falling off.

Moreover, the second core wire 70 is provided to be inserted into the first hollow shaft 10 through the opening 16 thereof and extended inside the first hollow shaft 10. In this manner, it is unnecessary to separately provide a hollow shaft through which the second core wire 70 is inserted. This makes the catheter 1 compact.

### [Second Embodiment]

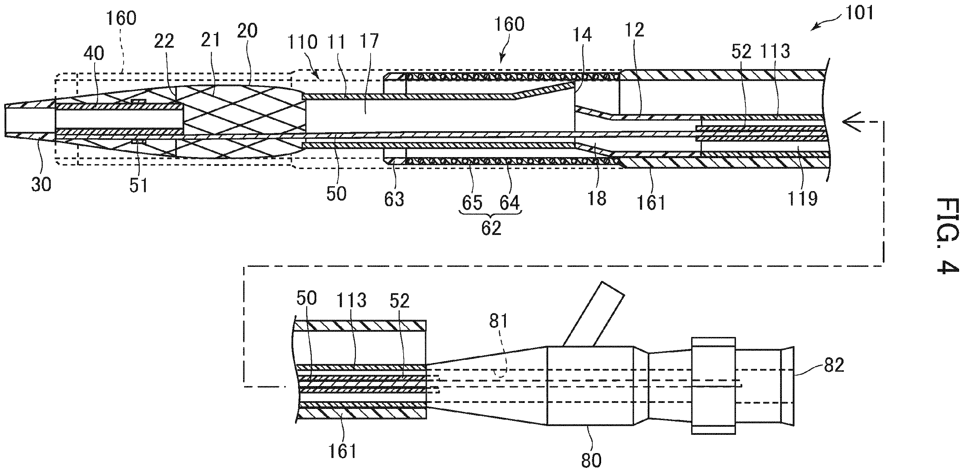

FIG. 4 is an overall view of a catheter 101 according to the second embodiment of the invention, illustrating a state in which a second hollow shaft 160 is located at the first position.

As illustrated in FIG. 4, the catheter 101 includes a first hollow shaft 110, the mesh member 20, the first distal end tip 30, the distal end hollow shaft 40, the first core wire 50, a second hollow shaft 160, and the controller 80. The catheter 101 of the second embodiment is different from the catheter 1 of the first embodiment in the aspect that the structures of the first hollow shaft 110 and the second hollow shaft 160 are different, and the aspect that the second core wire 70 is not provided. Note that the structures of the mesh member 20, the first distal end tip 30, the distal end hollow shaft 40, the first core wire 50, and the controller 80 are the same as those of the first embodiment. Thus, the same reference symbols are given to the same parts, and the detailed description thereof will be omitted. Further, the materials of the first hollow shaft 110 and the second hollow shaft 160 are the same as the materials of the first hollow shaft 10 and the second hollow shaft 60 of the first embodiment. Thus, the detailed description thereof will be omitted by referring to the description of the first embodiment.

The first hollow shaft 110 includes the distal end side shaft 11, the intermediate shaft 12, and a proximal end side shaft 113. The distal end side shaft 11 and the intermediate shaft 12 have the same structures as those of the first embodiment. The distal end of the proximal end side shaft 113 is connected to the proximal end of the intermediate shaft 12. The outer diameter and the inner diameter of the proximal end side shaft 113 are formed to be substantially equal to the outer diameter and the inner diameter of the intermediate shaft 12. Therefore, no opening is formed at the distal end of the proximal end side shaft 113. Only the first inner shaft 52 is fixed in the proximal end side shaft 113. Therefore, only the first core wire 50 is inserted through a lumen 119 of the proximal end side shaft 113.

The second hollow shaft 160 includes a proximal end portion 161, the intermediate portion 62, and the second distal end tip 63. The intermediate portion 62 and the second distal end tip 63 have the same structures as those of the first embodiment. The proximal end portion 161 is provided to surround the outer periphery of the first hollow shaft 10. In the second embodiment, the proximal end of the proximal end portion 161 extends to the proximal end of the proximal end side shaft 113 in a state where the second hollow shaft 160 is located at the first position. In the second embodiment, the operator pushes the proximal end of the proximal end portion 161 instead of the second core wire 70 so as to advance the second hollow shaft 160, whereby the second hollow shaft 160 is advanced from the first position to the second position (the position presented by dotted lines in FIG. 4).

In the catheter 101 of the second embodiment, the retrograde guide wire W2 is received by the mesh member 20, and the mesh member 20 is contracted so that the second hollow shaft 160 is moved from the first position to the second position. This allows the state where the second hollow shaft 160 surrounds the outer periphery of the mesh member 20 before the catheter 101 is removed. The catheter 101 is removed in this state, which makes it possible to prevent tissue pieces attached on the mesh member 20 from falling off.

### [Third Embodiment]

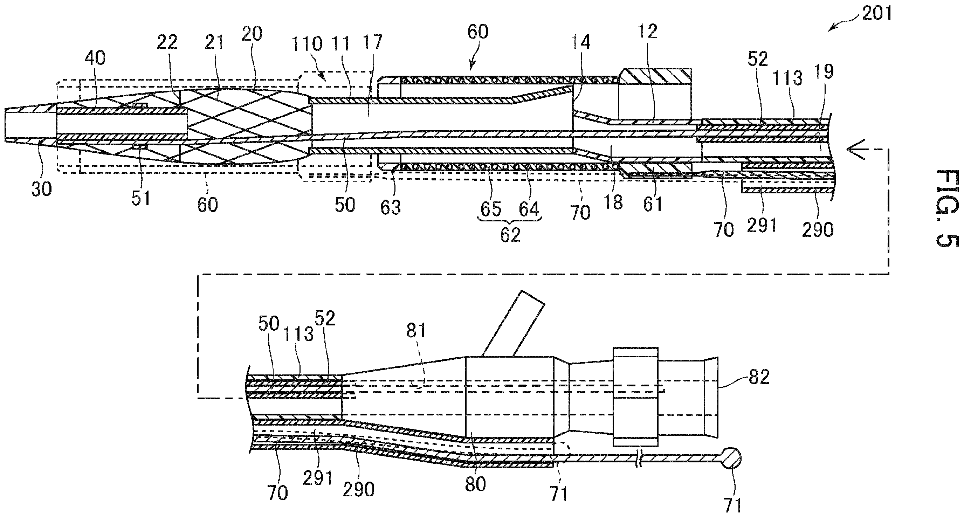

FIG. 5 is an overall view of a catheter 201 according to the third embodiment of the invention, illustrating a state in which the second hollow shaft 60 is located at the first position.

As illustrated in FIG. 5, the catheter 201 includes the first hollow shaft 110, the mesh member 20, the first distal end tip 30, the distal end hollow shaft 40, the first core wire 50, the second hollow shaft 60, the second core wire 70, the controller 80, and a third hollow shaft 290. The catheter 201 of the third embodiment is different from the catheter 1 of the first embodiment in the aspect that the structure of the first hollow shaft 110 is different, and the aspect that the third hollow shaft 290 is provided. Note that the structures of the mesh member 20, the first distal end tip 30, the distal end hollow shaft 40, the first core wire 50, and the controller 80 are the same as those of the first embodiment, and the structure of the first hollow shaft 110 is same as that of the second embodiment. Thus, the same reference symbols are given to the same parts, and the detailed description thereof will be omitted.

The third hollow shaft 290 is fixed to the outer surface of the proximal end side shaft 113 of the first hollow shaft 110. The distal end of the third hollow shaft 290 is positioned on the more proximal end side than the distal end of the proximal end side shaft 113, and the proximal end thereof extends to the controller 80. The third hollow shaft 290 includes a lumen 291 through which the second core wire 70 is inserted. The distal end of the second core wire 70 projects from the opening on the distal end side of the third hollow shaft 290, and is connected to the proximal end portion 61 of the second hollow shaft 60. As illustrated by dotted lines in FIG. 5, the protruding portion 71 of the second core wire 70 is formed to be in contact with the proximal end of the third hollow shaft 290 in the state where the second hollow shaft 60 is located at the second position. Examples of the material forming the third hollow shaft 290 include the same materials as those exemplified in the description of the first hollow shaft 10, for example.

In the catheter 201 of the third embodiment, the retrograde guide wire W2 is received by the mesh member 20, the mesh member 20 is contracted, and the second core wire 70 is pushed, so that the second hollow shaft 60 is moved from the first position to the second position. This allows the state where the second hollow shaft 60 surrounds the outer periphery of the mesh member 20 before the catheter 201 is removed. The catheter 201 is removed in this state, which makes it possible to prevent tissue pieces attached on the mesh member 20 from falling off.

### [Fourth Embodiment]

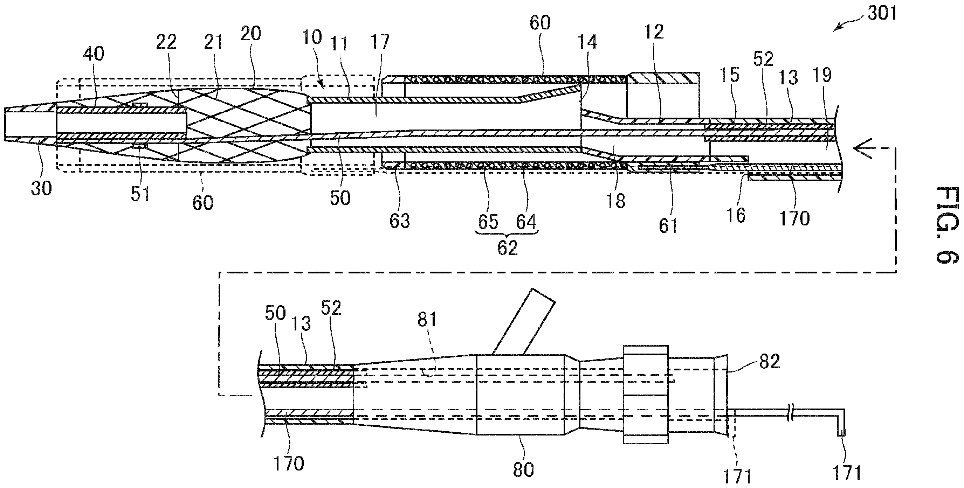

FIG. 6 is an overall view of a catheter 301 according to the third embodiment of the invention, illustrating a state in which the second hollow shaft 60 is located at the first position.

As illustrated in FIG. 6, the catheter 301 includes the first hollow shaft 10, the mesh member 20, the first distal end tip 30, the distal end hollow shaft 40, the first core wire 50, the second hollow shaft 60, a second core wire 170, and the controller 80. The catheter 201 of the third embodiment is different from the catheter 1 of the first embodiment in the aspect that the structure of the second core wire 170 is different. Note that the structures of the first hollow shaft 10, the mesh member 20, the first distal end tip 30, the distal end hollow shaft 40, the first core wire 50, the second hollow shaft 60, and the controller 80 are the same as those of the first embodiment. Thus, the same reference symbols are given to the same parts, and the detailed description thereof will be omitted. The material forming the second core wire 170 is the same as the material forming the second core wire 70 of the first embodiment. Thus, the detailed description thereof will be omitted by referring to the description of the first embodiment.

The distal end of the second core wire 170 is connected to the proximal end portion 61 of the second hollow shaft 60, and the proximal end thereof extends to be positioned on the more proximal end side than the proximal end of the controller 80. The distal end of the second core wire 170 is connected to be embedded in the proximal end portion 61. The second core wire 170 is provided to be inserted into the proximal end side shaft 13 of the first hollow shaft 10 through the opening 16 of the first hollow shaft 10 and extended inside the lumen 19 of the proximal end side shaft 13. A protruding portion 171 projecting outward in the radial direction of the controller 80 is provided at the proximal end of the second core wire 170. The protruding portion 171 is formed to be in contact with the proximal end of the controller 80 in the state where the second hollow shaft 60 is located at the second position, as illustrated by dotted lines in FIG. 6. In this manner, when the catheter 301 is removed, it is possible to prevent the second hollow shaft 60 from deviating from the outer periphery of the mesh member 20, and prevent tissue pieces attached on the mesh member 20 from falling off.

Note that the present invention is not limited to the structures of the above-described embodiments, but is defined by the terms of the claims and is intended to include any modifications within the scope and meaning equivalent to the terms of the claims. For example, a part of the structures of the above-described embodiments may be deleted or replaced by another structure, or another structure may be added to the structures of the above-described embodiments.

### Reference Signs List

- 1, 101, 201, 301: catheter
- 10, 110: first hollow shaft
- 16: opening
- 20: mesh member
- 30: distal end tip
- 50: first core wire
- 60, 160: second hollow shaft
- 70, 170: second core wire
- 71, 171: protruding portion
- 72: second inner shaft

## Claims

1. A catheter, comprising: a first hollow shaft;
a tubular mesh member that includes a proximal end joined to a distal end of the first hollow shaft and is formed to expand or contract in a radial direction;
a distal end tip that is joined to a distal end of the mesh member;
a first core wire that includes a distal end joined to the distal end tip and extends inside the mesh member and the first hollow shaft so that a proximal end of the first core wire is positioned on the more proximal end side than the proximal end of the first hollow shaft; and
a second hollow shaft that slides along an axial direction of the first hollow shaft and is movable between a first position at which the second hollow shaft surrounds an outer periphery of the first hollow shaft with a distal end of the second hollow shaft positioned on the more proximal side than the distal end of the first hollow shaft and a second position at which the second hollow shaft surrounds the outer periphery of the mesh member with the distal end of the second hollow shaft positioned on the more distal end side than the distal end of the first hollow shaft.

2. The catheter according to claim 1, further comprising a second core wire that includes a distal end joined to the second hollow shaft and a proximal end positioned on the more proximal end side than the proximal end of the first hollow shaft.

3. The catheter according to claim 2, wherein the first hollow shaft includes an opening communicating an outside and an inside of the first hollow shaft on the more proximal side than the second hollow shaft located at the first position, and
the second core wire is provided to be inserted into the first hollow shaft through the opening and extended inside the first hollow shaft.

4. The catheter according to claim 2 or 3, further comprising an inner shaft to which the second core wire is inserted, wherein
a protruding portion is provided at a proximal end of the second core wire, and
the protruding portion is formed to be in contact with the proximal end of the inner shaft when the second hollow shaft is located at the second position.

5. The catheter according to claim 2 or 3, wherein a protruding portion is provided at a proximal end of the second core wire, and
the protruding portion is formed to be in contact with the proximal end of the first hollow shaft when the second hollow shaft is located at the second position.
